# EUROPEAN PATENT SPECIFICATION

(11) **EP 4 475 950 B1**
(45) Date of publication and mention of the grant of the patent: **29.07.2026**
(21) Application number: 23703078.8
(22) Date of filing: 08.02.2023
(51) Int. Cl.: A61N 5/10

(54) **PROTON RADIOTHERAPY SYSTEM**
PROTONENSTRAHLENTHERAPIESYSTEM
SYSTÈME DE RADIOTHÉRAPIE DE PROTONS

(30) Priority: 09.02.2022 EP 22155954
(43) Date of publication of application: 18.12.2024
(73) Proprietor: Aarhus Universitet, 8000 Aarhus C (DK); Region Midtjylland, 8800 Viborg (DK)
(72) Inventor: RUGAARD POULSEN, Per, 8000 Aarhus C (DK); BREEDE BALTZER PETERSEN, Jørgen, 8800 Viborg (DK); VESTERGAARD, Anne, 8000 Aarhus C (DK); KALLEHAUGE, Jesper, 8000 Aarhus C (DK)
(74) Representative: Høiberg P/S
(86) International application number: PCT/EP2023/053097
(87) International publication number: WO 2023/152174

(56) References cited:
- EP-A1- 1 584 353
- US-A1- 2006 145 088
- US-A1- 2007 034 812

## Description

The present disclosure relates to a proton radiotherapy system and to a computer-implemented method of operating a proton radiotherapy system.

### Background

Different approaches are used in the treatment of cancer such as chemotherapy, surgery or radiotherapy, among others. Radiotherapy offers a non-invasive selective action in the tumor causing mild-to-moderate side effects in the healthy tissue nearby the tumor. Over the years, different types of treatment beams have been used in radiotherapy such as electrons, photons, ions and protons. Proton and ion beams offer interesting particle-matter interaction properties for their use on tumor treatment. One of the main advantages is the controllable tissue depth at which they deposit most of their energy, allowing to deliver the beam energy primarily in the tumor.

Therefore, a monoenergetic beam allows for depositing most of the particles' energy at one tissue depth, while a beam that uses a range of energies allows for depositing the particles' energy at a range of tissue depths, that correspond to the depths of the tumor. US 2007034812 A1 discloses methods of optimizing a laser-accelerated proton radiation dose to a targeted region. The method can be carried out either with the use of monoenergetic beams or the use of beams with a range of energies.

FLASH therapy is a promising novel radiotherapy technique for treating tumors. This therapy relies on the FLASH effect, which is a radiobiological phenomenon that reduces the radiation toxicity on healthy tissue compared to the irradiation received in standard radiotherapy, while still maintaining an anti-tumor response comparable to the standard therapy. This effect is triggered at ultra-high dose rates of ≥ 40 Gy/s and it may allow radiotherapy with a higher ratio between cure rate and toxicity rate. Radiotherapy with proton beams allows deposition of the beam dose primarily in the tumor while no dose is deposited in the healthy tissue placed beyond the spread-out Bragg peak. Proton FLASH therapy with ultra-high dose rates in the spread-out Bragg peak FLASH can be obtained using a beam degrader and ridge filter that are placed before the patient to alter the proton beam such that its energy dispersion is increased to treat a depth range of tumor tissue in the same exposure.

Nonetheless, treating specific tumors placed nearby critical radiosensitive organs, such as the eye nerve or the brain stem, might induce collateral irradiation in these critical areas with a high potentially harming result. The currently proposed methods for FLASH therapy involve beam pauses when delivering the FLASH therapy, which could hamper the FLASH effect.

Hence, advancements in FLASH therapy systems to improve the irradiation performance to address the above mentioned challenges would be beneficial.

### Summary

The invention is defined in the appended set of claims. Methods of radiotherapy described hereinafter do not form part of the present invention.

The present disclosure aims to solve at least one of existing challenges by providing a proton therapy system based on the FLASH effect for treating tumors. According to a first embodiment, a proton radiotherapy system is provided, which comprises:
- at least one radiation source comprising a proton accelerator, the at least one radiation source adapted to provide proton radiotherapy;
- a control unit for configuring the at least one radiation source in:
   ∘ a first configuration, wherein the at least one radiation source is configured to irradiate one or more sub-volumes and/or adjacent volumes of a target using at least one monoenergetic proton ultra-high dose rate beam; and
   ∘ a second configuration, wherein the at least one radiation source is configured to irradiate a remaining volume of the target, wherein the radiation is delivered using a range of energies.

The first radiation delivery configuration of the preferred embodiment may be referred to as 'FLASH painting'. The use of FLASH painting may be advantageous for treating tumors that are placed nearby or overlapping with healthy critical tissue of the patient, that needs to be protected from radiation.

By using a monoenergetic proton ultra-high dose rate beam in the one or more sub-volumes or adjacent volumes in the first configuration and covering the remaining volume of the target using non-ultra-high dose rate proton beams, a system is provided which provides efficient treatment of a total tumor volume close to a structure a risk with minimal effect to the structure at risk. Since the first configuration does not require a custom-made device placed between the accelerator gantry and the patient, the process is cost and time efficient.

The radiation source first configuration is characterized by the delivery of ultra-high proton dose rates in the order of ≥ 40 Gy/s with a single energy (monoenergetic). By using a monoenergetic proton beam in the first configuration, the energy is deposited primarily at a precise depth (the Bragg peak depth) depending on the initial energy of the beam and optionally on a range shifter. This is particularly beneficial in the interface region between the tumor and adjacent critical healthy tissue. The first configuration requires no custom-made devices placed between the accelerator gantry and the patient, making the process cost and time efficient. A standard range shifter, typically a relatively thick range shifter, may be used in the beam entrance path, to have a monoenergetic proton beam with an initial energy where an ultra-high dose rate is more easily obtained for the FLASH painting.

'Monoenergetic' in the context of the present disclosure may, as would be understood by a person skilled in the art, be construed to be substantially monoenergetic, i.e. may comprise a small energy dispersion. Moreover, using a monoenergetic proton ultra-high dose rate beam in the first configuration does not exclude that a further monoenergetic proton ultra-high dose rate beam of a different energy and/or a monoenergetic proton ultra-high dose rate beam delivered from a different direction is used after the first monoenergetic proton ultra-high dose rate beam. This may be the case, for example, if there are several sub-volumes or adjacent volumes to be covered by FLASH painting. Accordingly, in one embodiment of the presently disclosed proton radiotherapy system, the control unit is further configured to, in the first configuration, irradiate one or more sub-volumes or adjacent volumes of the target using further monoenergetic proton ultra-high dose rate beams. In one embodiment, the further monoenergetic proton ultra-high dose rate beams are used subsequently to the first monoenergetic proton ultra-high dose rate beam and/or wherein the further monoenergetic proton ultra-high dose rate beams are used on volumes that are different than the one or more sub-volumes or adjacent volumes of the target irradiated by the monoenergetic proton ultra-high dose rate beam.

A target may be a tumor. More specifically a target may be a gross tumor volume or a clinical target volume. A gross tumor volume (GTV) may be defined as what of the tumor can be seen, palpated or imaged. A clinical target volume (CTV) may be defined as a tissue volume that includes the gross tumor volume and subclinical malignant disease at a certain probability level. Alternatively, the CTV can be described as an extent of microscopic, un-imageable tumor spread. According to one embodiment of the presently disclosed radiotherapy system, the at least one radiation source may, in the first configuration, be configured to irradiate one or more sub-volumes or adjacent volumes of a target. A sub-volume may therefore be a volume within a gross tumor volume or a clinical target volume. An adjacent volume may refer to a volume that is not necessarily within the boundaries of the target but, for example, part of a radiotherapy planning target volume (PTV). The adjacent volume may even be outside the planning target volume. A planning target volume may be defined as a volume that encloses the clinical target volume with margins taking into account, for example, beam alignment, patient positioning, organ motion and organ deformation. The margin may be, for example, less than 20 mm, or less than 10 mm, depending on the circumstances, the target and the type of radiotherapy. As a person skilled in the art would understand, sub-volumes or adjacent volumes of a target shall also be construed to include any overlap between a sub-volumes and an adjacent volume. A person skilled in the art would also realize that 'adjacent volume' within the context of the present disclosure may depend on the circumstances. The person skilled in the art would nevertheless be capable of clearly understanding in which cases it would be useful to use FLASH painting. An adjacent volume may be, for example, a volume that is no longer than 50 mm from the target.

According to one embodiment, the system delivers a second beam configuration, which may deliver a non-ultra-high proton dose rates < 40 Gy/s. However, it is also possible to use FLASH, i.e. dose rates in the order of ≥ 40 Gy/s, in the second configuration. A typical dose rate at this configuration may be in the order of 1 Gy/s, as it is not required to trigger the FLASH effect. Any other suitable dose rate is possible and depends on the specific circumstances. The beam is preferably delivered using a range of proton energies. By using a proton beam with a range of energies, the radiation is delivered in a range of layers or depths (Bragg Peak depths), allowing to irradiate a series of depths in a tumor. A benefit of this configuration is the absence of beam modifying devices, such as a range shifter or a ripple filter, for the irradiation of the remaining part of the target not irradiated in the first configuration. This absence greatly reduces the by-product neutron generated by the proton interaction with the beam modifying devices, reducing the neutron radiation dose received by the patient and minimizing the probability of developing secondary tumors caused by the radiotherapy.

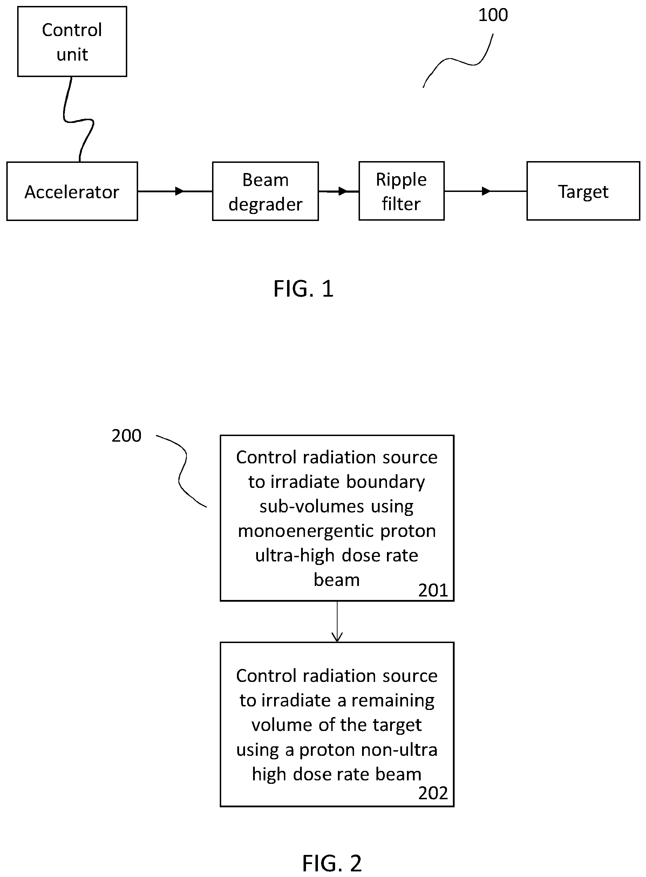

An example of a method of operating a proton radiotherapy system (200) is shown in fig. 2.

The presently disclosed approach may be particularly useful due to the combination of the first and second beam configurations. The first beam configuration allows to deliver radiation precisely at the tumor boundary while sparing healthy nearby tissue, whereas beam configuration two allows to reduce the by-product neutron radiation exposure of the patient during the radiotherapy and to obtain an overall high dose volume that conforms closely to the tumor shape. Furthermore, the disclosed approach may have a shorter irradiation time of by-product neutrons to the patient due to the presence of beam modifying devices during the first beam configuration, which may last in the order of ~ 100 ms. As well, the first beam configuration allows to precisely deliver the radiation nearby the critical tumor areas due to the short delivery time, avoiding spatial inaccuracies caused by patient movements and beam pauses.

In some embodiments, an accelerator system provides and delivers particle radiation comprised of He, C or Ne ions in the FLASH painting configuration.

Although certain preferred embodiments and configurations have been disclosed herein, it will be apparent to those skilled in the art that variations and modifications of such embodiments and configurations may be possible without substantially modifying the scope of the invention.

### Description of drawings

**Fig. 1** shows an embodiment of the presently disclosed radiotherapy system.
**Fig. 2** shows an embodiment computer-implemented method of operating a proton radiotherapy system.
**Fig. 3** shows an example of a planning process and delivery of the presently disclosed FLASH and non-FLASH delivery.
**Fig. 4** shows examples of beam spot path in the first configuration.
**Fig. 5** shows two examples of dose distributions.
**Fig. 6** shows a procedure for determining the fraction of dose delivered with FLASH and a FLASH weighted normal tissue dose.

All drawings are exemplary and not limiting to the presently disclosed radiotherapy system and method of operating a proton radiotherapy system.

### Detailed description

The present disclosure relates to a proton radiotherapy system comprising at least one radiation source comprising a proton accelerator, the at least one radiation source adapted to provide proton radiotherapy; and a control unit for controlling the at least one radiation source.

The system has a first configuration, wherein the at least one radiation source is configured to irradiate one or more sub-volumes or adjacent volumes of a target using a monoenergetic proton ultra-high dose rate beam. Preferably, the system also has a second configuration, wherein the at least one radiation source is configured to irradiate a remaining volume of the target using a non-ultra-high dose rate proton beam, wherein the radiation is delivered using a range of energies. Fig. 1 shows an example of a setup of the presently disclosed proton radiotherapy system (100). In the example the system comprises an accelerator, a beam degrader, a ripple filter and a control unit.

The technology may not only be used in proton radiotherapy systems but also in helium, carbon or neon ion particle radiotherapy system. Hence a further embodiment relates to a helium, carbon or neon ion particle radiotherapy system comprising:
- at least one radiation source comprising a helium, carbon or neon ion particle accelerator, the at least one radiation source adapted to provide helium, carbon or neon ion particle radiotherapy;
- a control unit for configuring the at least one radiation source in:
   ∘ a first configuration, wherein the at least one radiation source is configured to irradiate one or more sub-volumes and/or adjacent volumes of a target using at least one monoenergetic helium, carbon or neon ion particle ultra-high dose rate beam; and
   ∘ a second configuration, wherein the at least one radiation source is configured to irradiate a remaining volume of the target using a non-ultra-high dose rate helium, carbon or neon ion particle beam, wherein the radiation is delivered using a range of energies.

Proton therapy is a type of external beam radiotherapy that uses ionizing radiation. In proton therapy, medical personnel use a particle accelerator to target a tumor with a beam of protons. The proton radiotherapy accelerator may be, for example, synchrotron, cyclotron or superconducting synchrocyclotron, which is used to provide a beam of protons of the required energy and current. A person skilled in the art would, generally be able to set up and operate a proton radiotherapy system.

The FLASH effect requires an ultra-high dose rate in order to be triggered. Under this condition, FLASH effect has been demonstrated to preferentially spare healthy tissue, whereas damaging tumor cells without any sparing effect.

Previously reported FLASH radiotherapy plans have several disadvantages. One technique is the so-called spread-out Bragg peak FLASH, which requires the use of a thick standard range shifter and a custom-made 3D range modulator to modify the energy distribution of the beam of protons leaving the accelerator gantry. The physical interaction between the protons and the matter comprising the thick standard range shifter and a custom-made 3D range modulator generates by-product neutrons leading to an undesired irradiation to the patient. Due to the high spatial precision required to the proton beam, the custom-made 3D range modulator must be designed and machined specifically for each proton beam, increasing the clinical workload and costs per patient. Another disadvantage is that the dose in high-dose regions is delivered by several fields separated in time by pauses of the order of minutes. The beam pauses could easily hamper the FLASH effect.

In the preferred embodiment, a system comprising the proton accelerator may be configured to deliver proton radiation beams in two distinct configurations for treating tumors adjacent to or overlapping with critical healthy tissue. A first beam configuration delivered by the proton accelerator, irradiates one or more sub-volumes or adjacent volumes of a tumor target using one or more monoenergetic proton ultra-high dose rate beams, which may be referred to as 'FLASH painting'. FLASH painting may be adapted, although not restricted, to irradiate one or more tumor sub-volumes nearby critical healthy tissue. Due to relatively small extent of this surrounding area, compared with the total tumor volume, it is possible to use a quasi-monoenergetic beam. In this configuration, the embodiment may be configured to deliver a high proton energy beam of, for example, at least 200 MeV, such as ~ 250 MeV, to ensure the FLASH effect in the delivered dose, as cyclotron-based proton installations usually deliver the highest dose rates when operating at the highest acceleration energies. To reach a specific tumor depth, a standard range shifter may be placed in front of the patient to reduce the energy of the beam to the required value. Hence, in one embodiment, in the first configuration, the at least one radiation source is configured to irradiate one or more sub-volumes or adjacent volumes of a target using one or more monoenergetic proton ultra-high dose rate beam(s) obtained by a standard range shifter and/or a ridge filter. Typically, the depth at which the beam deposits the maximum energy is determined by a combination of the initial beam energy and the thickness and properties of the range shifter and/or a ridge filter. Therefore, in one embodiment, the initial beam energy is controlled to deposit a maximum of dose at a depth of the one or more sub-volumes or adjacent volumes. FLASH painting can be achieved in a range of treatment depths with a standard range shifter by adjusting the beam energy. The range shifter and/or a ridge filter is preferably arranged between an exit of the proton radiotherapy system and a subject.

A person skilled in the art would, generally, be able to select and control a non-custom-made device to adjust the deposited dose of the proton beam. Such non-custom-made devices include, but are not limited to, range shifters and ridge filters. As a non-limiting example, a range shifter can be composed by a body of water of ~ 30 cm thickness or other materials, such as a plastic body having the same water equivalent thickness. Other examples include graphite and boron carbide. A ripple filter comprising a sheet of material with thickness variations, such as plastic, inducing a small energy spread of the beam may be used for the treatment of sub-volumes or adjacent volumes larger thickness of > 1 cm. These examples of devices are not custom made and can be reused for different patients. The order of the devices placed after the proton beam gantry of the treatment room and the patient can be modified according to the requirements of the patient and characteristics of the described embodiment. A standard arrangement of the devices is disclosed as a beam degrader placed far from the patient and a ripple filter placed closer to the patient.

Hence, FLASH painting where custom-made proton beam modifying devices are not needed, is of great benefit for the clinical workflow and provides much wider flexibility for plan adaptation. In addition, neutron production and irradiation to the patient as well as device activation will be greatly reduced compared to previously proposed FLASH methods, as the thick standard range shifter is only used for a minor part of the treatment. This increases the quality of the delivered radiotherapy dose to the patient by delivering an uninterrupted ultra-high dose rate beam and reduces the costs without the custom-made devices specific for each patient.

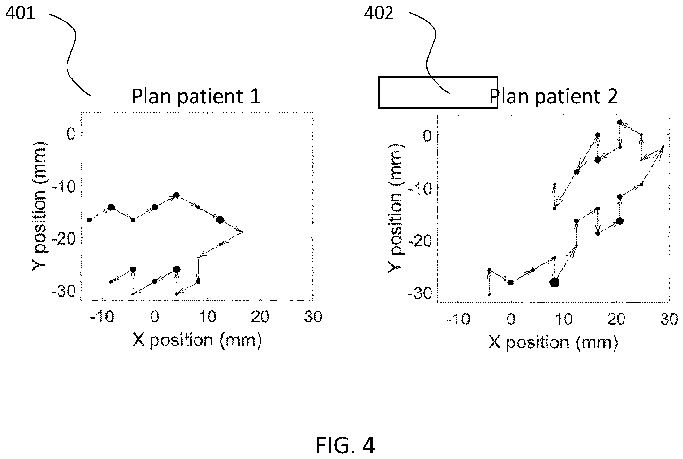

As stated above, in the first configuration, the at least one radiation source is configured to irradiate one or more sub-volumes or adjacent volumes of a target using a monoenergetic ultra-high dose rate proton beam. Preferably, the control unit is configured to move the at least one radiation source sequentially to irradiate a number of spots in the one or more sub-volumes or adjacent volumes in the first configuration. The sequence may comprise moving the beam in small steps to cover the whole sub-volume or adjacent volume of the target. Fig. 4 shows two examples (401; 402) of beam spot path in the first configuration. As can be seen, the control unit is configured to move the at least one radiation source sequentially in the x-y dimensions, i.e. the two dimensions perpendicular to the dimension corresponding to the depth z. The beam may be scanned following a path calculated by a computer-implemented method. The paths (401) and (402) are divided in the calculated dwell points where a specific dose is delivered. The spots in the figures are weighted to reflect the time that the beam stays in each position. The area comprising each dot is proportional to the delivered dose. In one embodiment of the presently disclosed proton radiotherapy system, the control unit is configured to move the at least one radiation source sequentially to irradiate a number of spots in the one or more sub-volumes in the boundary volume in the first configuration, wherein the radiation source stay in a number of predefined positions for a number of predefined periods of time. The control unit may be configured to move the at least one radiation source in time steps of less than 20 ms, preferably less than 10 ms, more preferably less than 1.0 ms, most preferably less than 0.3 ms. The time steps may refer to 'dwell times' where the beam resides in the positions as shown in fig. 4. The time spent in each spot will scale linearly with the delivered dose. This also means that the time steps will typically depend on the type of treatment. A total first configuration radiation delivery time for each sub-volume may be less than 1 s, preferably less than 500 ms, more preferably less than 200 ms. The at least one radiation source is configured to deliver a FLASH dose continuously, without pauses, in the first configuration. For the planning of the FLASH painting, it means that the control unit may be configured to move the at least one radiation source sequentially in an order that allows for continuous delivery of FLASH. Preferably, the control unit is configured to move the at least one radiation source in spot steps of less than 10 mm.

In a further embodiment, the proton radiotherapy system may be configured to irradiate distal edges of the target with respect to the position of the at least one radiation source in the first configuration. It may be an advantage to apply FLASH painting in these areas. The range of a given proton beam in a given subject is normally not known exactly. The consequences of this range uncertainty could be mitigated by irradiating the distal edge of the target with ultra-high dose rates in the first configuration. If the range of the protons is longer than anticipated the high dose delivered to normal tissue in the distal edge of the proton beam will be less harmful due to the FLASH effect. The treatment will therefore be more robust to range uncertainties. Moreover, proton beams have a higher radiobiological effect at the distal edge of the beam where the linear energy transfer is higher. It means that the same dose may be more damaging to normal tissue if it is delivered by the distal edge of the beam. This problem could also be mitigated by irradiating the distal edge of the target with ultra-high dose rates in the first configuration.

There are typically no strict limits on the volumes of target and sub-volumes and adjacent volumes of the target. The presently disclosed proton radiotherapy system may be used on different types of tumors, structures at risk and overlapping or adjacent sub-volumes. The one or more sub-volumes may be, for example, less than 10 cm³, preferably less than 5 cm³, more preferably less than 2 cm³. The structures at risk may be, for example, brain stem, optical nerves, chiasm, spinal cord, duodenum or urethra.

In one embodiment of the presently disclosed proton radiotherapy system a second beam configuration is used to irradiate a remaining volume of the tumor target using one or more proton beam comprised of a range of energies. In the second configuration there may typically be a plurality of proton beams, such as 2-4 beams. The beams may be arranged to irradiate from different directions. This configuration may deliver radiation at dose rate of less than 40 Gy/s (non-FLASH) without the necessity of beam modifying devices, avoiding undesired neutron radiation to the patient generated by the interaction between the proton beam and these devices. However, it is also possible to use FLASH, i.e. dose rates in the order of ≥ 40 Gy/s, in the second configuration. Using FLASH as an option in the second configuration may involve, but is not limited to, going through a sequence of different beam energies to cover at least a substantial part, such at least half of, the target. The range of energies of the proton beam may be selected directly by controlling accelerators according to the tumor depth that needs to be irradiated. The second configuration may have a significantly longer radiation delivery time than the first configuration. According to one embodiment the second configuration radiation delivery time is longer than 10 s, preferably between 10 s and 5 minutes or 10 minutes. The second configuration may comprise a plurality of beams. In the second configuration, the system may be arranged to irradiate the whole volume of the target, for example, by irradiating in three directions. Comparing the dose delivery time of both configurations, it is apparent that FLASH painting requires a considerably smaller time compared with the second configuration. This greatly enhances the spatial precision on the delivery of the FLASH painting in the areas where the sparing effect is mostly needed and reduces the exposure to by-product neutrons to a minimum amount compared to the amount that would be generated otherwise with previously reported proton FLASH configurations.

Recent studies suggest the use of He, C or Ne ion particles in radiotherapy showing a comparable behavior than protons, with an improved outcome on the tumor elimination. The system and the method of operating the system disclosed herein can be extrapolated by using the mentioned ion particles. Hence, the present disclosure further relates to a helium, carbon or neon ion particle radiotherapy system comprising:
- at least one radiation source comprising a helium, carbon or neon ion particle accelerator, the at least one radiation source adapted to provide helium, carbon or neon ion particle radiotherapy;
- a control unit for configuring the at least one radiation source in:
   ∘ a first configuration, wherein the at least one radiation source is configured to irradiate one or more sub-volumes or adjacent volumes of a target using at least one monoenergetic helium, carbon or neon ion particle ultra-high dose rate beam; and
   ∘ a second configuration, wherein the at least one radiation source is configured to irradiate a remaining volume of the target using a non-ultra-high dose rate helium, carbon or neon ion particle beam, wherein the radiation is delivered using a range of energies.

A person skilled in the art would recognize the all features of the proton radiotherapy system may be used interchangeably be the helium, carbon or neon ion particle radiotherapy system.

The present disclosure further relates to a computer-implemented method of operating a proton radiotherapy system. An example of such a method is shown in fig. 2. The method (200) comprises the steps of:
- in a first configuration, controlling a radiation source to irradiate one or more boundary sub-volumes of a target tumor using a monoenergetic proton ultra-high dose rate beam (201);
- in a second configuration, controlling the radiation source to irradiate a remaining volume of the target tumor using a proton non ultra-high dose rate beam, wherein the radiation is delivered using a range of energies (202).

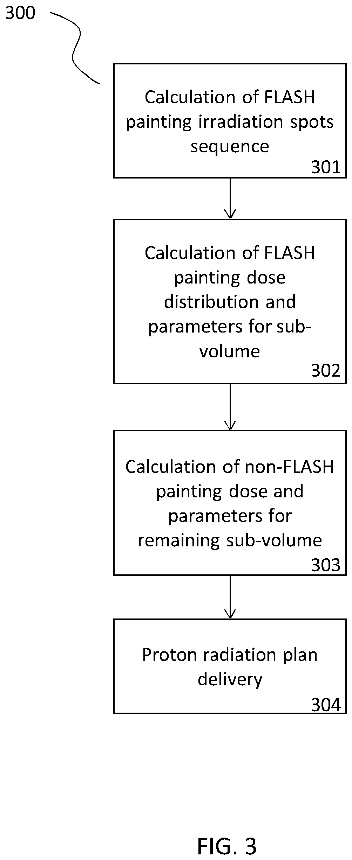

Fig. 3 shows an example (300) of how the presently disclosed system and method may operate. First, a FLASH painting configuration plan (301) of the radiotherapy system is calculated, based on the tumor location, type and adjacent critical organs. The calculated plan characteristics (302) typically include beam conditions such as energy and current provided by the accelerator, the specific material and dimensions of a range shifter and a ripple filter for the system configuration that requires them, a beam pattern specifying a number and location of irradiation points location in the tumor, the beam irradiation duration in each spot, the dwell time between irradiation spots, the radiation energy delivered in each point and the total dose delivered in the tumor. In the next step the plan characteristics for the second configuration is calculated (303), such as beam conditions, energy and current provided by the accelerator, a beam pattern specifying a number and location of irradiation points location in the tumor, the beam irradiation duration in each spot, the dwell time between irradiation spots, the radiation energy delivered in each point and the total dose delivered in the tumor. Finally, the computer-implemented method operates the radiotherapy system and delivers the calculated plan (304).

### Example, quantitative study of FLASH painting

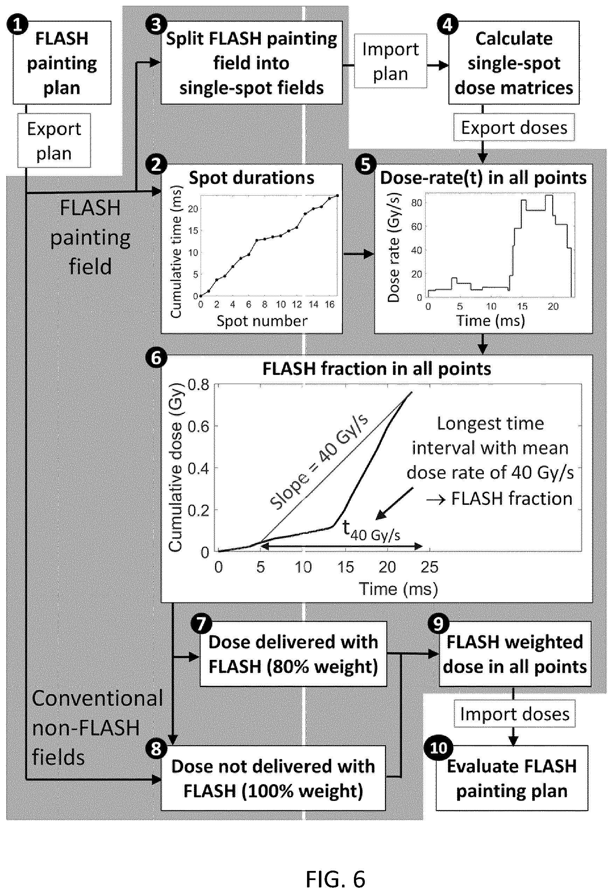

Fig. 6 may be referred to as a quantitative study of FLASH painting and the presently disclosed proton radiotherapy system and computer-implemented method of operating a proton radiotherapy system. Delivery of the proton FLASH painting field was simulated for two proton FLASH painting treatment plans. First, each plan was exported from the treatment planning system (TPS) (fig. 6, L1) and the spot sequence of the FLASH painting field was rearranged to avoid beam pauses. Log files with the spot durations were retrieved and the fastest possible unconstrained field delivery was estimated by scaling the spot durations from the actual cyclotron current to the maximum available cyclotron current of 800nA (L2). Next, the dose distribution of each spot was determined by splitting the FLASH painting field into single-spot fields (L3), which were imported and recalculated in the TPS (L4). The dose and duration of each spot were combined to yield the instantaneous dose rate as function of time in all TPS calculation points (L5). Time integration of the dose rate resulted in the cumulative dose as function of time (L6).

To quantify the fraction of dose delivered under FLASH conditions (the "FLASH fraction") it may be assumed that a FLASH effect is triggered in a point when the dose is delivered continuously with a mean dose rate of at least 40Gy/s. A further assumption may be a FLASH sparing effect of 20% for normal tissue, i.e., that the dose delivered under FLASH conditions has a radiobiological effect on normal tissue that corresponds to 80% of the dose. These assumptions enabled illustration of the proton FLASH painting concept through quantitative analysis. For each TPS calculation point, the dose delivered under FLASH conditions (D_{40Gy/s}) was calculated as follows for the FLASH painting field. For points with a maximum instantaneous dose rate below 40Gy/s, D_{40Gy/s} was set to zero. For points with an average dose rate above 40Gy/s for the entire FLASH painting field, D_{40Gy/s} was set equal to the dose from the FLASH painting field. For all other points, the longest time interval t_{40Gy/s} with a mean dose rate of 40Gy/s was found from the cumulative dose as function of time (L6). D_{40Gy/s} was then calculated as t_{40Gy/s}x40Gy/s. Next, the "FLASH weighted" dose (L9) was calculated as D_{40Gy/s} weighted by 80% (L7) plus the remaining dose weighted by 100% (L8). The dose with and without FLASH weighting was finally compared in the TPS to illustrate the potential brainstem sparing effect of proton FLASH painting (L10).

### Examples

In order to illustrate some of the presently disclosed features of proton radiotherapy system, the following examples are provided:
Therapy for an IDH-1 mutated anaplastic astrocytoma (Patient-1) and an anaplastic oligodendroglioma (Patient-2). The clinical target volume (CTV) was 177cm³ for Patient-1 and 128cm³ for Patient-2.

For both patients, an IMPT FLASH painting plan was made consisting of three conventional IMPT fields with the same gantry and couch angles as the clinical plan supplemented with a single-energy FLASH painting field from one of the three original field directions. For the FLASH painting field, a range shifter of ~31cm water equivalent thickness was modelled in the TPS by a standard 5cm range shifter supplemented with a structure of 26cm water equivalent thickness. The range shifter allowed for a higher beam energy and thus a higher potential dose rate.

Field specific spot placement volumes, in which the plan optimizer could place pencil beam Bragg peaks, were used to drive the plan optimization towards a solution where the FLASH painting field delivered nearly all dose to a FLASH painting volume where normal tissue sparing was particularly important. For Patient-1, the spot placement volume for the FLASH painting field was the brainstem-CTV overlap volume extended with manually defined margins of up to 1cm. For Patient-2, it was the CTV volume closest to the brainstem extended with manually defined margins that avoided brainstem overlap.

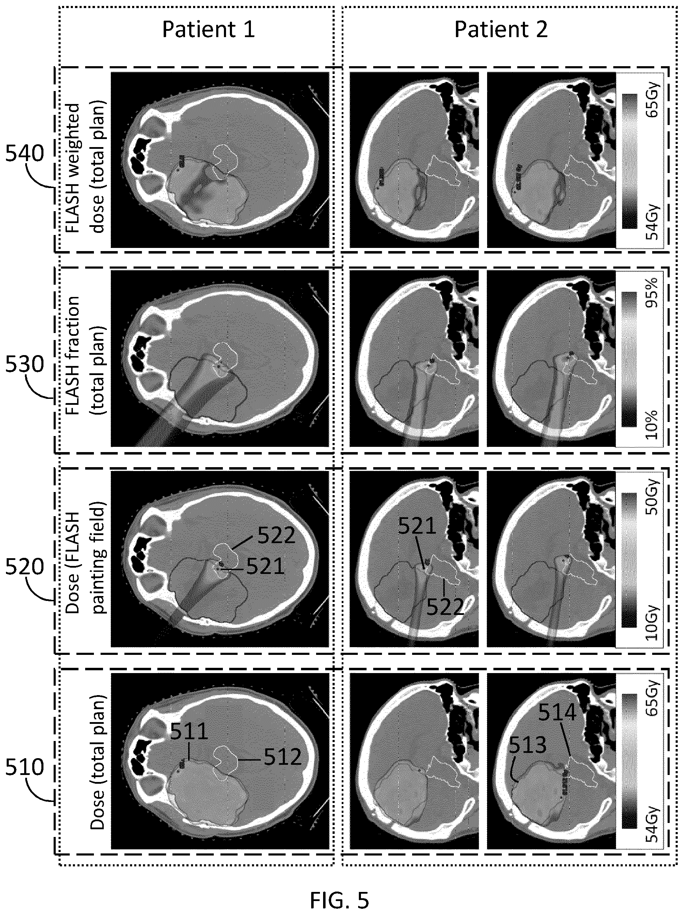

Fig. 5 shows dose distributions in an axial plane for Patient-1 and a sagittal place for Patient-2. Fig. 5 shows dose distribution for total plan (510) and the FLASH painting field (520), fraction of the total dose delivered continuously with a mean dose rate of at least 40Gy/s (530) and FLASH weighted dose distribution for the total plan (540). In the bottom left and bottom right drawings the target (511; 513) is subject to a radiotherapy plan. A structure at risk (512, 514) is located adjacent to or overlaps the target (511; 513). In the FLASH painting field (520) row in fig. 5, the sub-volume (521) is a sub-volume adjacent to or overlapping a structure at risk (522).

The total dose plan (510) shows a homogeneous irradiation of the tumor in both patients. The FLASH painting field was applied in the tumor areas nearby the brain stem, as depicted in (520), and it represented a 5 - 7 % of the total irradiation for a duration of ~ 200 ms. The fraction of the total dose comprised of FLASH radiation is represented in (530). The FLASH weighted dose (540) is calculated by multiplying by a factor of 0.8 the dose delivered in FLASH painting mode plus the sum of the dose delivered in the second configuration. The factor of 0.8 is considered to be the sparing effect of the radiation in the healthy tissue in FLASH conditions. The FLASH weighted dose is used for visualizing the radiation distribution in the second configuration, taking into account the sparing effect of the first configuration.

In the example of fig.5, Patient-1 had V90%=100%, V95%=99.9% and V107%=0.07%. Patient-2 had V90%=100% (99.1-100% across robustness scenarios), V95%=99.99% (98.2-99.7%) and V107%=0.04% (0-0.8%). The FLASH painting fields mainly delivered dose in or near the brainstem (520).

For Patient-1, 1.92cm³ of the brainstem received more than 50Gy. The FLASH fraction in this high dose volume was 17.2-84.6% (530) and the FLASH weighted dose was 83.1-96.6% of the dose. FLASH weighting reduced brainstem V54Gy from 1.54cm³ to 0.01cm³ (540).

For Patient-2, the brainstem volume receiving more than 45Gy was 0.20cm³ for the nominal plan and 1.09cm³ for the worst-case robustness scenario with highest brainstem dose. The FLASH fraction in these volumes was 40.6-89.5% (nominal plan, 530) and 17.5-90.1% (worst-case scenario, 530). The FLASH weighted doses were 82.1-91.9% (nominal plan) and 82.0-96.5% (worst-case scenario) of the dose. FLASH weighting reduced brainstem V54Gy from 0.33cm³ to 0.02cm³ for the worst-case scenario (540).

Table 1 provides a summary of the setup. Delivery simulations with maximum cyclotron current (800nA) gave FLASH field durations of 22.9-24.8ms with calculation point dependent instantaneous dose rates up to 184Gy/s and field averaged dose rates up to 64Gy/s.

**Table 1. Characteristics of the proton FLASH painting fields**

| | Patient-1 | Patient-2 |
|---|---|---|
| Proton beam energy | 232.5 MeV | 235.3 MeV |
| Number of spots | 17 | 23 |
| MU fraction of whole plan | 7.7% | 5.0% |
| Cyclotron current (experiment) | 76.5 nA | 66.4 nA |
| Field duration (experiment) | 239 ms | 300 ms |
| Field duration at 800nA (simulation) | 22.9 ms | 24.8 ms |
| Spot duration at 800nA (simulation) | 0.26 - 3.3ms | 0.22 - 4.6ms |
| Highest instantaneous dose rate in a point | 146 Gy/s | 184 Gy/s |
| Highest field averaged dose rate in a point | 63.9 Gy/s | 61.2 Gy/s |

## Claims

1. A proton radiotherapy system (100) comprising:
- at least one radiation source comprising a proton accelerator, the at least one radiation source adapted to provide proton radiotherapy;
- a control unit for configuring the at least one radiation source in:
∘ a first configuration, wherein the at least one radiation source is configured to irradiate one or more sub-volumes and/or adjacent volumes of a target using at least one monoenergetic proton ultra-high dose rate beam; and
∘ a second configuration, wherein the at least one radiation source is configured to irradiate a remaining volume of the target, wherein the radiation is delivered using a range of energies.

2. The proton radiotherapy system (100) according to any one of the preceding claims, wherein the at least one radiation source is configured to deliver radiation at dose rate greater than 40 Gy/s, FLASH, in the first configuration.

3. The proton radiotherapy system (100) according to any one of the preceding claims, wherein the control unit is configured to move the at least one radiation source sequentially to irradiate a number of spots in the one or more sub-volumes or adjacent volumes of the target in the first configuration.

4. The proton radiotherapy system (100) according to any one of the preceding claims, wherein a first configuration radiation delivery time is less than 1 s, preferably less than 500 ms, more preferably less than 200 ms.

5. The proton radiotherapy system (100) according to any one of the preceding claims, wherein the at least one radiation source is configured to deliver a FLASH dose continuously, without pauses, in the first configuration.

6. The proton radiotherapy system (100) according to any of claims 3-5, wherein the control unit is configured to move the at least one radiation source sequentially in an order that allows for continuous delivery of FLASH.

7. The proton radiotherapy system (100) according to any one of the preceding claims, wherein the system is configured to irradiate at least one distal edge of the target with respect to the position of the at least one radiation source in the first configuration.

8. The proton radiotherapy system (100) according to any one of the preceding claims, wherein, in the first configuration, the at least one radiation source is configured to irradiate one or more sub-volumes or adjacent volumes of the target of a target using a monoenergetic proton ultra-high dose rate beam obtained by a standard range shifter and/or a ridge filter.

9. The proton radiotherapy system (100) according to any one of the preceding claims, wherein, in the second configuration, the control unit controls the proton accelerator to deliver a range of energies and to deposit the energies in the target.

10. The proton radiotherapy system (100) according to any one of the preceding claims, wherein the one or more sub-volumes or adjacent volumes of the target is adjacent to and/or overlapping a structure at risk, such as a brainstem volume.

11. The proton radiotherapy system (100) according to any one of the preceding claims, wherein the one or more sub-volumes or adjacent volumes of the target are less than 10 cm³, preferably less than 5 cm³, more preferably less than 2 cm³.

12. The proton radiotherapy system (100) according to any one of the preceding claims, wherein control unit is further configured to, in the first configuration, irradiate one or more sub-volumes or adjacent volumes of the target using further monoenergetic proton ultra-high dose rate beams.

13. A computer program having instructions which, when executed by a computing device or computing system, cause the computing device or computing system to carry out a computer-implemented method of operating a proton radiotherapy system (100) comprising the steps of:
- in a first configuration, controlling a radiation source to irradiate one or more boundary sub-volumes and/or adjacent volumes of a target using at least one monoenergetic proton ultra-high dose rate beam; and
- in a second configuration, controlling the radiation source to irradiate a remaining volume of the target, wherein the radiation is delivered using a range of energies.

14. A helium, carbon or neon ion particle radiotherapy system comprising:
- at least one radiation source comprising a helium, carbon or neon ion particle accelerator, the at least one radiation source adapted to provide helium, carbon or neon ion particle radiotherapy;
- a control unit for configuring the at least one radiation source in:
∘ a first configuration, wherein the at least one radiation source is configured to irradiate one or more sub-volumes and/or adjacent volumes of a target using at least one monoenergetic helium, carbon or neon ion particle ultra-high dose rate beam; and
∘ a second configuration, wherein the at least one radiation source is configured to irradiate a remaining volume of the target, wherein the radiation is delivered using a range of energies.

## Patentansprüche

1. Protonenstrahlentherapiesystem (100) umfassend:
• mindestens eine Strahlungsquelle, die einen Protonenbeschleuniger umfasst, wobei die mindestens eine Strahlungsquelle zur Bereitstellung von Protonenstrahlentherapie ausgebildet ist;
• eine Steuereinheit zur Konfiguration der mindestens einen Strahlungsquelle in:
∘ einer ersten Konfiguration, wobei die mindestens eine Strahlungsquelle dazu konfiguriert ist, ein oder mehrere Teilvolumina und/oder benachbarte Volumina eines Ziels unter Verwendung mindestens eines monoenergetischen Protonen-Ultrahochdosisratenstrahls zu bestrahlen; und
∘ einer zweiten Konfiguration, wobei die mindestens eine Strahlungsquelle dazu konfiguriert ist, ein verbleibendes Volumen des Ziels zu bestrahlen, wobei die Strahlung unter Verwendung eines Energiebereichs abgegeben wird.

2. Protonenstrahlentherapiesystem (100) nach einem der vorangehenden Ansprüche, wobei die mindestens eine Strahlungsquelle dazu konfiguriert ist, in der ersten Konfiguration Strahlung mit einer Dosisrate größer als 40 Gy/s, FLASH, abzugeben.

3. Protonenstrahlentherapiesystem (100) nach einem der vorangehenden Ansprüche, wobei die Steuereinheit dazu konfiguriert ist, die mindestens eine Strahlungsquelle sequenziell zu bewegen, um eine Anzahl von Spots in den ein oder mehreren Teilvolumina oder benachbarten Volumina des Ziels in der ersten Konfiguration zu bestrahlen.

4. Protonenstrahlentherapiesystem (100) nach einem der vorangehenden Ansprüche, wobei eine Strahlungsabgabezeit der ersten Konfiguration weniger als 1 s beträgt, vorzugsweise weniger als 500 ms, noch bevorzugter weniger als 200 ms.

5. Protonenstrahlentherapiesystem (100) nach einem der vorangehenden Ansprüche, wobei die mindestens eine Strahlungsquelle dazu konfiguriert ist, in der ersten Konfiguration eine FLASH-Dosis kontinuierlich, ohne Pausen, abzugeben.

6. Protonenstrahlentherapiesystem (100) nach einem der Ansprüche 3-5, wobei die Steuereinheit dazu konfiguriert ist, die mindestens eine Strahlungsquelle sequenziell in einer Reihenfolge zu bewegen, die eine kontinuierliche Abgabe von FLASH ermöglicht.

7. Protonenstrahlentherapiesystem (100) nach einem der vorangehenden Ansprüche, wobei das System dazu konfiguriert ist, mindestens eine distale Kante des Ziels in Bezug auf die Position der mindestens einen Strahlungsquelle in der ersten Konfiguration zu bestrahlen.

8. Protonenstrahlentherapiesystem (100) nach einem der vorangehenden Ansprüche, wobei in der ersten Konfiguration die mindestens eine Strahlungsquelle dazu konfiguriert ist, ein oder mehrere Teilvolumina oder benachbarte Volumina des Ziels eines Ziels unter Verwendung eines monoenergetischen Protonen-Ultrahochdosisratenstrahls zu bestrahlen, der durch einen Standard-Reichweitenverschieber und/oder einen Rillenfilter erhalten wird.

9. Protonenstrahlentherapiesystem (100) nach einem der vorangehenden Ansprüche, wobei in der zweiten Konfiguration die Steuereinheit den Protonenbeschleuniger steuert, um einen Energiebereich abzugeben und die Energien im Ziel zu deponieren.

10. Protonenstrahlentherapiesystem (100) nach einem der vorangehenden Ansprüche, wobei die ein oder mehreren Teilvolumina oder benachbarten Volumina des Ziels an eine Struktur mit Risiko angrenzen und/oder diese überlappen, wie etwa ein Hirnstammvolumen.

11. Protonenstrahlentherapiesystem (100) nach einem der vorangehenden Ansprüche, wobei die ein oder mehreren Teilvolumina oder benachbarten Volumina des Ziels kleiner als 10 cm³ sind, vorzugsweise kleiner als 5 cm³, noch bevorzugter kleiner als 2 cm³.

12. Protonenstrahlentherapiesystem (100) nach einem der vorangehenden Ansprüche, wobei die Steuereinheit ferner dazu konfiguriert ist, in der ersten Konfiguration ein oder mehrere Teilvolumina oder benachbarte Volumina des Ziels unter Verwendung weiterer monoenergetischer Protonen-Ultrahochdosisratenstrahlen zu bestrahlen.

13. Computerprogramm mit Anweisungen, die, wenn sie von einem Rechengerät oder einem Rechnersystem ausgeführt werden, das Rechengerät oder das Rechnersystem veranlassen, ein computerimplementiertes Verfahren zum Betreiben eines Protonenstrahlentherapiesystems (100) auszuführen, das die Schritte umfasst:
• in einer ersten Konfiguration Steuern einer Strahlungsquelle, um ein oder mehrere Rand-Teilvolumina und/oder benachbarte Volumina eines Ziels unter Verwendung mindestens eines monoenergetischen Protonen-Ultrahochdosisratenstrahls zu bestrahlen; und
• in einer zweiten Konfiguration Steuern der Strahlungsquelle, um ein verbleibendes Volumen des Ziels zu bestrahlen, wobei die Strahlung unter Verwendung eines Energiebereichs abgegeben wird.

14. Helium-, Kohlenstoff- oder Neon-Ionenstrahlentherapiesystem umfassend:
• mindestens eine Strahlungsquelle, die einen Helium-, Kohlenstoff- oder Neon-Ionenpartikelbeschleuniger umfasst, wobei die mindestens eine Strahlungsquelle zur Bereitstellung von Helium-, Kohlenstoff- oder Neon-lonenpartikelstrahlentherapie ausgebildet ist;
• eine Steuereinheit zur Konfiguration der mindestens einen Strahlungsquelle in:
∘ einer ersten Konfiguration, wobei die mindestens eine Strahlungsquelle dazu konfiguriert ist, ein oder mehrere Teilvolumina und/oder benachbarte Volumina eines Ziels unter Verwendung mindestens eines monoenergetischen Helium-, Kohlenstoff- oder Neon-lonenpartikel-Ultrahochdosisratenstrahls zu bestrahlen; und
∘ einer zweiten Konfiguration, wobei die mindestens eine Strahlungsquelle dazu konfiguriert ist, ein verbleibendes Volumen des Ziels zu bestrahlen, wobei die Strahlung unter Verwendung eines Energiebereichs abgegeben wird.

## Revendications

1. Système de radiothérapie protonique (100) comprenant :
• au moins une source de rayonnement comprenant un accélérateur de protons, la au moins une source de rayonnement étant adaptée pour fournir une radiothérapie protonique;
• une unité de commande pour configurer la au moins une source de rayonnement en:
∘ une première configuration, dans laquelle la au moins une source de rayonnement est configurée pour irradier un ou plusieurs sous-volumes et/ou volumes adjacents d'une cible en utilisant au moins un faisceau de protons monoénergétique à débit de dose ultra-élevé ; et
∘ une seconde configuration, dans laquelle la au moins une source de rayonnement est configurée pour irradier un volume restant de la cible, dans laquelle le rayonnement est délivré en utilisant une gamme d'énergies.

2. Système de radiothérapie protonique (100) selon l'une quelconque des revendications précédentes, dans lequel la au moins une source de rayonnement est configurée pour délivrer un rayonnement à un débit de dose supérieur à 40 Gy/s, FLASH, dans la première configuration.

3. Système de radiothérapie protonique (100) selon l'une quelconque des revendications précédentes, dans lequel l'unité de commande est configurée pour déplacer la au moins une source de rayonnement de manière séquentielle afin d'irradier un nombre de points dans le ou les sous-volumes ou volumes adjacents de la cible dans la première configuration.

4. Système de radiothérapie protonique (100) selon l'une quelconque des revendications précédentes, dans lequel un temps de délivrance du rayonnement de la première configuration est inférieur à 1 s, de préférence inférieur à 500 ms, plus préférablement inférieur à 200 ms.

5. Système de radiothérapie protonique (100) selon l'une quelconque des revendications précédentes, dans lequel la au moins une source de rayonnement est configurée pour délivrer une dose FLASH de manière continue, sans pauses, dans la première configuration.

6. Système de radiothérapie protonique (100) selon l'une quelconque des revendications 3-5, dans lequel l'unité de commande est configurée pour déplacer la au moins une source de rayonnement de manière séquentielle dans un ordre qui permet une délivrance continue de FLASH.

7. Système de radiothérapie protonique (100) selon l'une quelconque des revendications précédentes, dans lequel le système est configuré pour irradier au moins un bord distal de la cible par rapport à la position de la au moins une source de rayonnement dans la première configuration.

8. Système de radiothérapie protonique (100) selon l'une quelconque des revendications précédentes, dans lequel, dans la première configuration, la au moins une source de rayonnement est configurée pour irradier un ou plusieurs sous-volumes ou volumes adjacents de la cible d'une cible en utilisant un faisceau de protons monoénergétique à débit de dose ultra-élevé obtenu par un déplaceur de portée standard et/ou un filtre à crêtes.

9. Système de radiothérapie protonique (100) selon l'une quelconque des revendications précédentes, dans lequel, dans la seconde configuration, l'unité de commande commande l'accélérateur de protons pour délivrer une gamme d'énergies et pour déposer les énergies dans la cible.

10. Système de radiothérapie protonique (100) selon l'une quelconque des revendications précédentes, dans lequel le ou les sous-volumes ou volumes adjacents de la cible sont adjacents à et/ou chevauchent une structure à risque, telle qu'un volume du tronc cérébral.

11. Système de radiothérapie protonique (100) selon l'une quelconque des revendications précédentes, dans lequel le ou les sous-volumes ou volumes adjacents de la cible sont inférieurs à 10 cm³, de préférence inférieurs à 5 cm³, plus préférablement inférieurs à 2 cm³.

12. Système de radiothérapie protonique (100) selon l'une quelconque des revendications précédentes, dans lequel l'unité de commande est en outre configurée pour, dans la première configuration, irradier un ou plusieurs sous-volumes ou volumes adjacents de la cible en utilisant d'autres faisceaux de protons monoénergétiques à débit de dose ultra-élevé.

13. Programme informatique comportant des instructions qui, lorsqu'elles sont exécutées par un dispositif informatique ou un système informatique, amènent le dispositif informatique ou le système informatique à réaliser un procédé mise en œuvre par ordinateur de fonctionnement d'un système de radiothérapie protonique (100) comprenant les étapes de:
• dans une première configuration, commander une source de rayonnement pour irradier un ou plusieurs sous-volumes frontières et/ou volumes adjacents d'une cible en utilisant au moins un faisceau de protons monoénergétique à débit de dose ultra-élevé; et
• dans une seconde configuration, commander la source de rayonnement pour irradier un volume restant de la cible, dans laquelle le rayonnement est délivré en utilisant une gamme d'énergies.

14. Système de radiothérapie par particules d'ions hélium, carbone ou néon comprenant:
• au moins une source de rayonnement comprenant un accélérateur de particules d'ions hélium, carbone ou néon, la au moins une source de rayonnement étant adaptée à fournir une radiothérapie par particules d'ions hélium, carbone ou néon;
• une unité de commande pour configurer la au moins une source de rayonnement en:
∘ une première configuration, dans laquelle la au moins une source de rayonnement est configurée pour irradier un ou plusieurs sous-volumes et/ou volumes adjacents d'une cible en utilisant au moins un faisceau de particules d'ions hélium, carbone ou néon monoénergétique à débit de dose ultra-élevé; et
∘ une seconde configuration, dans laquelle la au moins une source de rayonnement est configurée pour irradier un volume restant de la cible, dans laquelle le rayonnement est délivré en utilisant une gamme d'énergies.
